# EUROPEAN PATENT APPLICATION

(11) **EP 4 696 725 A1**
(43) Date of publication of application: **18.02.2026**
(21) Application number: 24868476.3
(22) Date of filing: 09.08.2024
(51) Int. Cl.: C08F 226/06, C08F 290/06, C08F 4/34, C07D 251/30

(54) **CURING COMPOSITION AND CURABLE COMPOSITION COMPRISING SAME**

(30) Priority: 22.09.2023 KR 20230127289; 09.07.2024 KR 20240090506
(71) Applicant: Kolon Industries, Inc., Seoul 07793 (KR)
(72) Inventor: SEO, Da Young, Seoul 07793 (KR); CHUNG, Kun Ho, Seoul 07793 (KR); NAM, Sae Rom, Seoul 07793 (KR); HUANG, Guang Chun, Seoul 07793 (KR); KANG, Sung Kyu, Seoul 07793 (KR)
(74) Representative: Vossius & Partner Patentanwälte Rechtsanwälte mbB
(86) International application number: PCT/KR2024/011875
(87) International publication number: WO 2025/063497

(57) **Abstract**

Provided is a curing agent composition including: a first compound represented by Formula 1; and a second compound represented by Formula 2. When the curing agent composition is used, dielectric characteristics, chemical resistance, and high-temperature stability may be improved.

## Description

### TECHNICAL FIELD

The present disclosure relates to a curing agent composition and a curing composition including the same.

### BACKGROUND ART

Recently, as the volume of the information and communications increases, information and communications in high-frequency bands are actively practiced. Accordingly, the need for materials that can respond to high-frequency regions is increasing. In particular, materials with a low dissipation factor are being required to reduce transmission loss in high-frequency bands, and materials that can achieve high heat resistance while having a dissipation factor of 0.005 or less at least 10 GHz are being required.

In particular, triallyl isocyanurate (TAIC) was mainly used as a curing agent for high-frequency copper-clad laminations (CCLs) to implement excellent low dielectric characteristics. The TAIC is crosslinked with a polymer via double bonds present at the terminus by an initiator, and the polymer is cured, thereby providing high-frequency CCLs with low dielectric characteristics.

However, when the TAIC is crosslinked under vacuum and high temperature conditions, partial volatilization of some of the TAIC occurs due to a low decomposition temperature (Td) of the TAIC, resulting in curing deviations at different sites. In this regard, problems with heat resistance and durability are caused.
Accordingly, to prevent the occurrence of curing deviations, the need for a curing agent composition having low dissipation factor characteristics as well as a high decomposition temperature Td is increasing.

### DESCRIPTION OF EMBODIMENTS

### TECHNICAL PROBLEM

The technical problem of the present disclosure is to provide a curing composition having low dissipation factor characteristics as well as a high decomposition temperature.

### SOLUTION TO PROBLEM

An aspect of the present disclosure relates to a curing agent composition including: a first compound represented by Formula 1; and a second compound represented by Formula 2:

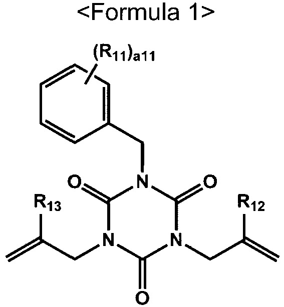

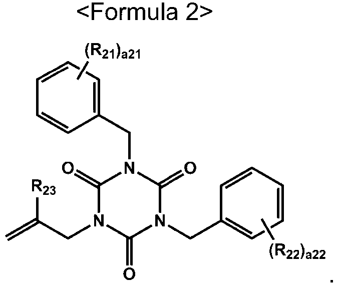

In Formulae 1 and 2,
R₁₁ to R₁₃ and R₂₁ to R₂₃ may each independently be hydrogen, a C₁-C₁₅ alkyl group, or a C₂-C₁₅ alkenyl group, and
a11, a21, and a22 may each independently be an integer from 0 to 5.

Another aspect of the present disclosure relates to a curing composition including the curing agent composition.

### ADVANTAGEOUS EFFECTS OF DISCLOSURE

A curing agent composition according to the present disclosure has a bulkier benzyl group than an allyl group, and thus, as the size of a network structure formed by using the curing agent composition increases, the size of voids increases. Accordingly, an article prepared by using the curing agent composition may have improved dielectric characteristics.

In addition, as the curing agent composition includes both a first compound and a second compound at the same time, chemical resistance, high-temperature stability, and mechanical characteristics of the curing agent composition may be improved .

### MODE OF DISCLOSURE

Hereinafter, various aspects and various embodiments of the present disclosure are described in more detail.

Terms or words as used in the present specification and claims not to be construed as limited to ordinary or dictionary meaning, but should be interpreted as having meanings and concepts consistent with the technical idea of the disclosure, based on the principle that the inventor can appropriately define the concept of the terms to explain the disclosure of the inventor in the best method.

Terms as used in the present disclosure are for the purpose of describing particular embodiments only, and are not intended to limit the present disclosure. An expression used in the singular encompasses the expression of the plural, unless it has a clearly different meaning in the context. It will be further understood that the terms "comprises" or "includes" when used in the present specification, specify the presence of stated features, integers, steps, operations, elements, components, or combinations thereof, but do not preclude the presence or addition of one or more other features, integers, steps, operations, elements, components, or combinations thereof.

In detail, a curing agent composition according to an aspect of the present disclosure includes: a first compound represented by Formula 1; and a second compound represented by Formula 2:

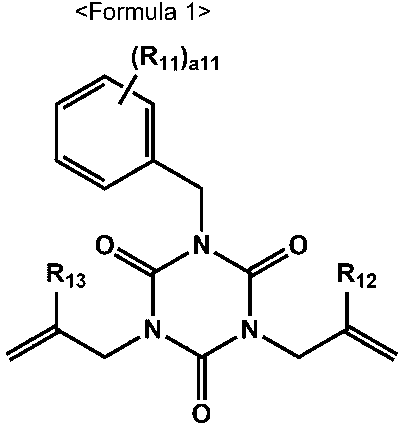

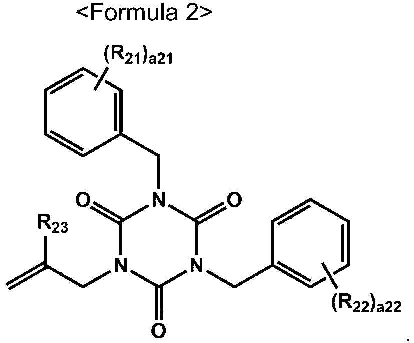

In Formulae 1 and 2,
R₁₁ to R₁₃ and R₂₁ to R₂₃ may each independently be hydrogen, a C₁-C₁₅ alkyl group, or a C₂-C₁₅ alkenyl group, and
a11, a21, and a22 may each independently be an integer from 0 to 5.

In an embodiment, the curing agent composition may have a 5 % decomposition temperature Td of 150 °C or more. For example, the decomposition temperature Td refers to the temperature at which the mass of a sample is reduced by 5 % based on the results of thermogravimetric analysis (TGA) measurement. For example, the curing agent composition may have a decomposition temperature Td in a range of 150 to 240 °C, 160 °C to 240 °C, 170 °C to 240 °C, or 180 °C to 240 °C. For example, when the decomposition temperature Td of the curing agent composition satisfies the ranges above, chemical resistance and high-temperature stability of an article prepared by using the curing agent composition may be improved.

In an embodiment, the amount of the first compound may be in a range of 70 to 99 weight% based on the total amount of the curing agent composition, and the amount of the second compound may be in a range of 1 to 30 weight% based on the total amount of the curing agent composition.

In an embodiment, the amount of the first compound may be, based on the total weight of the curing agent composition, in a range of 80 to 99 weight%, 85 to 99 weight%, or 90 to 99 weight.

In an embodiment, the amount of the second compound may be, based on the total weight of the curing agent composition, in a range of 1 to 20 weight%, 1 to 15 weight%, or 1 to 10 weight%.

In an embodiment, in Formula 1, R₁₁ to R₁₃ may each independently be hydrogen or a C₁-C₁₅ alkyl group.

In an embodiment, in Formula 1, R₁₁ to R₁₃ may each independently be hydrogen, a methyl group, or an ethyl group.

In an embodiment, the first compound may be represented by Formula 1-1:

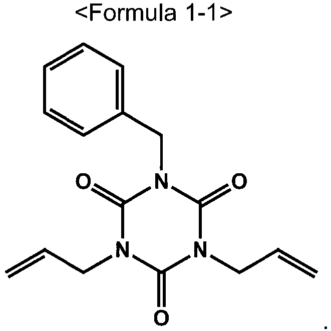

In an embodiment, in Formula 2, R₂₁ to R₂₃ may each independently be hydrogen or a C₁-C₁₅ alkyl group.

In an embodiment, in Formula 2, R₂₁ to R₂₃ may each independently be hydrogen, a methyl group, or an ethyl group.

In an embodiment, the second compound may be represented by Formula 2-1:

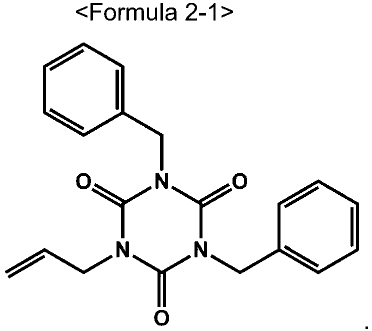

In an embodiment, the curing agent composition may further include a third compound represented by Formula 3:

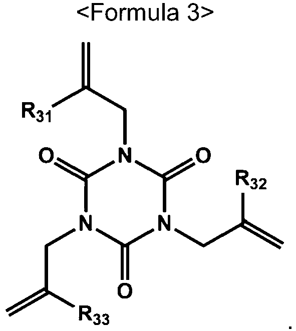

In Formula 3,
R₃₁ to R₃₃ may each independently be hydrogen, a C₁-C₁₅ alkyl group, or a C₂-C₁₅ alkenyl group.

In an embodiment, in Formula 3, R₃₁ to R₃₃ may each independently be hydrogen or a C₁-C₁₅ alkyl group.

In an embodiment, in Formula 3, R₃₁ to R₃₃ may each independently be hydrogen, a methyl group, or an ethyl group.

In an embodiment, the third compound may be represented by Formula 3-1:

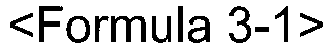

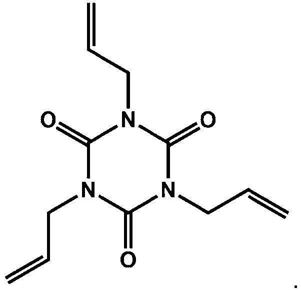

In an embodiment, the amount of the third compound may be 30 weight% or less based on the total weight of the curing agent composition. For example, the amount of the third compound may be, based on the total weight of the curing agent composition, in a range of 1 to 25 weight%, 1 to 20 weight%, 1 to 15 weight%, or 1 to 10 weight%.

In an embodiment, the curing agent composition may further include a fourth compound represented by Formula 4:

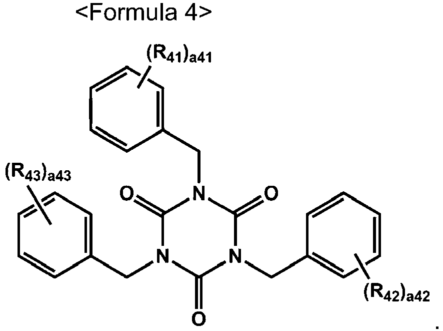

In Formula 4,
R₄₁ to R₄₃ may each independently be hydrogen, a C₁-C₁₅ alkyl group, or a C₂-C₁₅ alkenyl group, and
a41, a42, and a43 may each independently be an integer from 0 to 5.

In an embodiment, in Formula 4, R₄₁ to R₄₃ may each independently be hydrogen or a C₁-C₁₅ alkyl group.

In an embodiment, in Formula 4, R₄₁ to R₄₃ may each independently be hydrogen, a methyl group, or an ethyl group.

In an embodiment, the fourth compound may be represented by Formula 4-1:

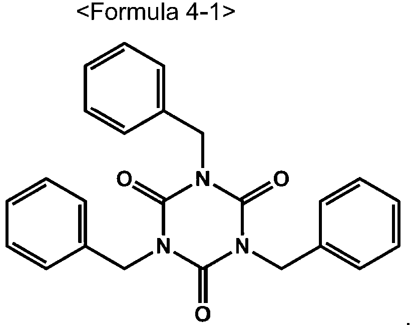

In an embodiment, the amount of the fourth compound in the curing agent composition may be, based on the total weight of the curing agent composition, 5 weight% or less, 4 weight% or less, 3 weight% or less, 2 weight% or less, or 1 weight% or less.

A curing composition according to another aspect of the present disclosure includes: the curing agent composition; a base polymer; and an initiator.

The base polymer may be, for example, crosslinked with a neighboring base polymer via bonding with double bonds included in the curing agent composition.

In an embodiment, the base polymer may include a modified polyphenylene oxide (mPPO). For example, the mPPO may include a polyphenylene oxide modified with acrylate, methacrylate, an aryl group, or a vinyl group.

In an embodiment the mPPO may be represented by Formula 5:

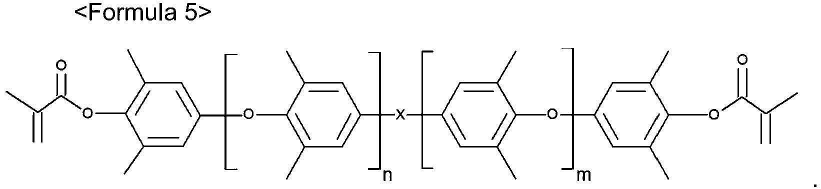

In Formula 5, n and m may each independently be an integer of 1 or more, and X may be a C₁-C₁₅ alkylene group or a C₂-C₁₅ alkenylene group. For example, in Formula 5, X may be a methylene group, an ethylene group, an n-propylene group, an isopropylene group, or a butylene group.

The initiator may initiate a crosslinking reaction between the curing agent composition and the base polymer.

In an embodiment, the initiator may include a peroxide-based initiator.

In an embodiment, the peroxide-based initiator may include dicumyl peroxide, diacylperoxide, 2,4-dichlorobenzoyl peroxide, isobutyryl peroxide, decanoyl peroxide, lauryl peroxide, propionyl peroxide, acetyl peroxide, benzoyl peroxide, p-chlorobenzoyl peroxide, or any combination thereof.

In an embodiment, the initiator may include dicumyl peroxide.

In an embodiment, a ratio of the number of moles of the initiator to the number of moles of the curing agent composition may be in a range of 0.01 to 0.5. For example, the ratio of the number of moles of the initiator to the number of moles of the curing agent composition may be in a range of 0.05 to 0.5, 0.1 to 0.5, 0.1 to 0.4, or 0.2 to 0.4.

In an embodiment, the curing composition may further include a solvent.

In an embodiment, the solvent may include toluene, acetone, methyl ethyl ketone, ethyl acetate, or any combination thereof.

In an embodiment, the solvent may include toluene.

In an embodiment, in the curing composition, the amount of the base polymer included may be in a range of 50 to 90 weight% based on the total weight of the solids, the amount of the curing agent composition may be in a range of 1 to 35 weight% based on the total weight of the solids, and the amount of the initiator may be in a range of 1 to 15 weight% based on the total weight of the solids. The total weight of the solids may refer to the total weight of the components, except for the solvent, included in the curing composition.

In an embodiment, a ratio of the weight of the base polymer to the weight of the curing agent composition may be in a range of 0.1 to 10. For example, the ratio of the weight of the base polymer to the weight of the curing agent composition may be in a range of 1 to 10, 2 to 10, 1 to 8, 1 to 6, 1 to 4, or 1 to 3.

In an embodiment, the curing composition may have a glass transition temperature Tg of 260 °C or less. For example, the glass transition temperature Tg of the curing agent composition may be in a range of 180 to 260 °C, 180 to 250 °C, 180 to 245 °C, or 200 to 245 °C.

In an embodiment, the curing composition may have a dielectric constant Dk of 2.55 or less. For example, the curing agent composition may a dielectric constant Dk in a range of 2 to 2.55, 2 to 2.5, 2 to 2.4, or 2.2 to 2.3.

In an embodiment, the curing composition may have a dissipation factor Df of 0.0044 or less. For example, the curing agent composition may have a dissipation factor Df in a range of 0.002 to 0.0044, 0.002 to 0.004, 0.002 to 0.0038, or 0.0025 to 0.0035.

### [Definition of terms]

In the present specification, the C₁-C₁₅ alkyl group refers to a linear or branched aliphatic hydrocarbon monovalent group that has 1 to 15 carbon atoms, and specific examples thereof may include a methyl group, an ethyl group, an *n*-propyl group, an isopropyl group, an *n*-butyl group, a *sec*-butyl group, an isobutyl group, a *tert*-butyl group, an *n*-pentyl group, a *tert*-pentyl group, a neopentyl group, an isopentyl group, a *sec*-pentyl group, a 3-pentyl group, a *sec*-isopentyl group, an *n*-hexyl group, an isohexyl group, a *sec*-hexyl group, a *tert*-hexyl group, an *n*-heptyl group, an isoheptyl group, a *sec*-heptyl group, a *tert*-heptyl group, an *n*-octyl group, an isooctyl group, a *sec*-octyl group, a *tert-*octyl group, an *n*-nonyl group, an isononyl group, a sec-nonyl group, a *tert*-nonyl group, an *n*-decyl group, an isodecyl group, a *sec*-decyl group, and a *tert-*decyl group. In the present specification, the C₁-C₁₅ alkylene group refers to a linear or branched aliphatic divalent hydrocarbon group having 1 to 15 carbon atoms.

In the present specification, the C₂-C₁₅ alkenyl group refers to a monovalent hydrocarbon group having at least one carbon-carbon double bond in the middle or at the terminus of the C₂-C₁₅ alkyl group, and examples thereof may include an ethenyl group, a propenyl group, a butenyl group, and the like. The C₂-C₁₅ alkenylene group refers to a divalent hydrocarbon group having at least one carbon-carbon double bond in the middle or at the terminus of the C₂-C₁₅ alkylene group.

Hereinafter, the present disclosure will be described in more detail through examples. These examples are to explain the present disclosure in more detail, and the scope of the present disclosure is not limited by these examples, and this will be apparent to those skilled in the art to which the present disclosure belongs.

### Preparation Example 1. Preparation of curing agent composition

### Preparation of diallyl-isocyanurate (DAIC)

15.0 parts by mass of cyanuric acid, 120.0 parts by mass of water, 0.12 parts by mass of CuCl, and 27.9 parts by mass of 50 % NaOH were added to a 5-necked flask equipped with a thermometer, a condenser, and a stirrer, and the mixture was stirred.

18.67 parts by mass of allyl chloride was added to the mixture, and after raising the temperature to 45 °C, a reaction was allowed for 1 hour. After cooling the resulting mixture to 30 °C, 4.56 parts by mass of phosphoric acid was added to the mixture to precipitate DAIC.

The precipitated DAIC was filtered, washed with 30 parts by mass of water and 30 parts by mass of methanol, and then, dried in a vacuum oven at 100 °C for 12 hours, thereby obtaining 13 parts by mass of dried DAIC (purity of 96 %, pale blue powder).

### <DAIC>

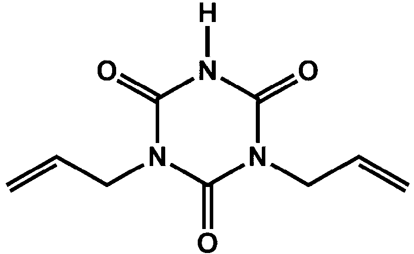

### Preparation of curing agent composition

10.0 parts by mass of DAIC, 15.0 parts by mass of DMF, and 3.90 parts by mass of 50 % NaOH were added to a 5-necked flask equipped with a thermometer, a condenser, and a stirrer, and the mixture was stirred.

6.17 parts by mass of benzyl chloride was added to the mixture, and after raising the temperature to 100 °C, a reaction was allowed for 1 hour. After cooling the mixture to 30 °C, the produced NaCl was filtered through a paper filter, and the filtrate was added back to the 5-necked flask and then concentrated under reduced pressure at 100 °C.

To the flask where the concentration was complete, a washing process was performed thereon by adding 30 parts by mass of ethyl acetate (EA) and 15 parts by mass of water, and was performed again twice more by adding 15 parts by mass of water.

Afterwards, the organic layer in the flask was filtered through 2.0 parts by mass of diatomite, and the filtrate was washed with 5 parts by mass of EA. Afterwards, the reaction product was concentrated under reduced pressure under a temperature condition of 70 °C, 10 parts by mass of methanol was added to the concentrated product which was then concentrated again under reduced pressure under a temperature condition of 10 °C to be recrystallized. Powder thus produced was filtered, and the filtrate was washed with 10 parts by mass of methanol and dried in a vacuum oven at 45 °C for 12 hours, thereby obtaining 9 parts by mass of a curing agent composition (purity of 96 %, white powder).

The amount ratios of a first compound, a second compound, and a third compound in the curing agent composition are shown in Table 1.

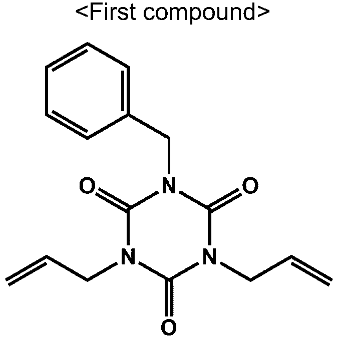

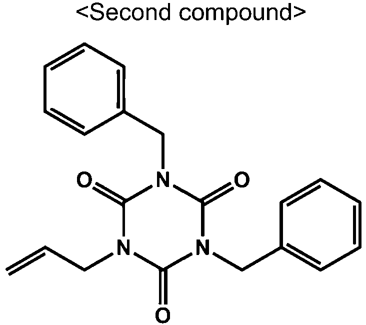

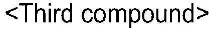

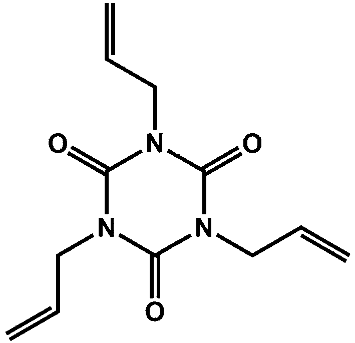

### Preparation Examples 2 to 8. Preparation of curing agent composition

A curing agent composition was prepared in the same manner as in Preparation Example 1, except that the amount ratios of the first compound, the second compound, and the third compound in the curing agent composition was changed as shown in Table 1 by adjusting the input amounts of the reactants.

### Preparation Examples 9 to 11. Preparation of curing agent composition

A curing agent composition was prepared in the same manner as in Preparation Example 1, except that, after preparing DAIC, the first compound and the second compound were separated by column separation (hexane:EA=1:1) and then the first compound and the second Compound were mixed at a ratio as shown in Table 1.

### Comparative Preparation Example 1. Preparation of second compound

In the presence of 58.18 g (0.42 mol) of potassium carbonate in 205 g of dimethylformamide, 35.6 g (0.21 mol) of allyl isocyanurate and 33.68 g (0.42 mol) of benzyl chloride were allowed for a reaction at 100 °C for 2 hours. Afterwards, following cooling, the reaction mixture was filtered to remove inorganic substances, the filtrate was distilled under reduced pressure to recover the solvent, and the residue was diluted with chloroform, and then was subjected to extraction to extract alkali aqueous solution and acid aqueous solution. The extract thus obtained was washed, dried with anhydrous magnesium sulfate, and filtered, and chloroform in the filtrate was distilled under reduced pressure to recover. Isopropyl alcohol was added to the residue to precipitate and filter the crystals. The cake thus obtained was dried to prepare a curing agent composition including a second compound (purity of 96 %).

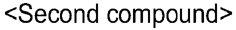

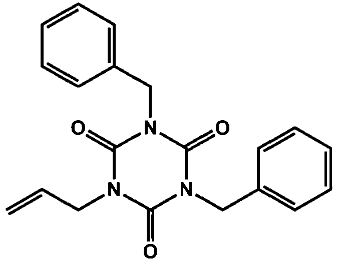

### Comparative Preparation Example 2. Preparation of third compound

A curing agent composition including a third compound was prepared by using triallyl isocyanurate (TAIC, product number: 114235) available from Sigma-aldrich Company.

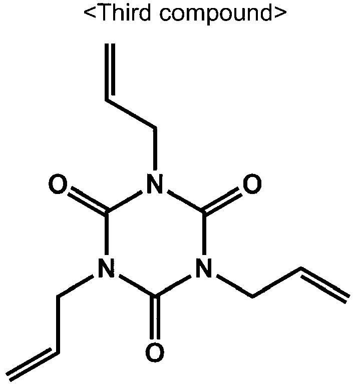

### Evaluation Example 1: Measurement of decomposition temperature Td

By using a thermogravimeter analyzer (TGA), the temperature was raised at a rate of 20 °C per minute under purge gas N₂ conditions up to a maximum of 900 °C, and the 5% loss Td (°C) of the curing agent compositions prepared according to Preparation Examples 1 to 11 and Comparative Preparation Examples 1 and 2. Results of the measurement are shown in Table 1.

**[Table 1]**

| | Curing agent composition | | | |
|---|---|---|---|---|
| | First compound | Second compound | Third compound | 5 % loss Td (°C) |
| Preparation Example 1 | 96 | 2 | 2 | 184.3 |
| Preparation Example 2 | 90 | 8 | 2 | 186.4 |
| Preparation Example 3 | 90 | 5 | 5 | 184.5 |
| Preparation Example 4 | 85 | 13 | 2 | 188.9 |
| Preparation Example 5 | 85 | 5 | 10 | 182.0 |
| Preparation Example 6 | 85 | 2 | 13 | 180.1 |
| Preparation Example 7 | 80 | 18 | 2 | 190.7 |
| Preparation Example 8 | 60 | 16 | 24 | 181.5 |
| Preparation Example 9 | 95 | 5 | - | 186.6 |
| Preparation Example 10 | 90 | 10 | - | 188.7 |
| Preparation Example 11 | 85 | 15 | - | 189.4 |
| Comparative Preparation Example 1 | - | 100 | - | 220.8 |
| Comparative Preparation Example 2 | - | - | 100 | 146.7 |

Referring to Table 1, the curing agent compositions prepared according to Preparation Examples had an increased decomposition temperature Td by including the first compound and the second compound. Accordingly, the curing agent compositions according to Preparation Examples had excellent high-temperature stability.

### Example 1

5.0 parts by mass of the curing agent (the curing agent composition of Preparation Example 1) and 20.64 parts by mass of toluene as a solvent were added to 20.0 parts by mass of polyphenylene oxide modified with methacrylate (KPU-6000), and the mixture was dissolved by using a sonicator for 2 hours (at a maximum temperature of 40 °C). Afterwards, 0.226 parts by mass of dicumyl peroxide (DCP) as an initiator was added to the mixture to prepare varnish.

Glass fibers were impregnated in the varnish, dried at room temperature for 10 minutes, and then dried by heating at 130 °C for 3 minutes. After grinding the dried curing product into powder, the powder was poured into a mold having a size of length x width x height of 1.5 cm x 1.5 cm x 0.5 cm. Afterwards, the mold containing the powder of the curing product was placed between copper foils. The copper foils were compressed for 2 hours at 70 °C at 300 bar by using a pressurizing press, and the temperature was raised at 230 °C and maintained for 2 hours. After the pressure was removed, the copper foils and the mold were removed to obtain a pressured curing product. The pressed curing product was cut into a size of width of 1cm and length of 1 cm to prepare a specimen.

### Examples 2 to 11 and Comparative Examples 1 and 2

A specimen was prepared in the same manner as in Example 1, except that the amount of the solvent, the type of the curing agent, the amount of the curing agent, and the amount of the initiator were changed as shown in Table 2.

### Evaluation Example 2: Measurement of dielectric constant Dk and dissipation factor Df

The dielectric constant Dk at 10 GHz of the specimens of Examples 1 to 11 and Comparative Examples 1 and 2 was measured by using a measuring device (ADMS01O Series by AET Company).

To measure the dielectric constant Dk of the specimens of Examples 1 to 11 and Comparative Examples 1 and 2, the frequency of the measuring device was selected as 9.3 GHz. The frequencies of two reference materials were input for the calibration measurement. The calibration was performed by selecting, as the reference materials, PTFE (Dk=2.03) and SiO₂ (Dk=4.41).

First, as the blank, the dielectric constant Dk of the air was measured (Dk = 1). After completion of the blank measurement, the dielectric constant Dk of PTFE selected first as the reference material was measured. After placing the specimen on a probe, a vacuum pump was turned on and a measuring button was pressed to measure the dielectric constant Dk.

After completion of the PTFE measurement, the dielectric constant Dk of SiO₂ selected second as the reference material was measured. After completion of the calibration, the dielectric constant Dk of PTFE and SiO₂ selected as the reference materials was measured. When the error range between the actual value and the measured value was beyond ±1%, the calibration was performed again.

Afterwards, each of the specimens according to Examples 1 to 11 and Comparative Examples 1 and 2 was placed on a probe. Then, a vacuum pump was turned on to prepare for maximum compression to prepare the dielectric constant Dk. The dielectric constant Dk values and the dissipation factor Df values were calculated from the dielectric constant measurement results, and are shown in Table 2.

**[Table 2]**

| | KPU-6000 | Toluene | DCP | Curing agent | | Tg (°C) | Dielectric constant (Dk) | Dissipation factor (Df) |
|---|---|---|---|---|---|---|---|---|
| | | | | Type | Amount | | | |
| Example 1 | 20 | 20.64 | 0.226 | Preparation Example 1 | 5.0 | 235 | 2.3013 | 0.0028 |
| Example 2 | 20 | 20.64 | 0.226 | Preparation Example 2 | 5.0 | 229.1 | 2.3014 | 0.0028 |
| Example 3 | 20 | 20.64 | 0.226 | Preparation Example 3 | 5.0 | 232.9 | 2.3082 | 0.0029 |
| Example 4 | 20 | 20.64 | 0.226 | Preparation Example 4 | 5.0 | 223.5 | 2.3149 | 0.0029 |
| Example 5 | 20 | 20.64 | 0.226 | Preparation Example 5 | 5.0 | 240.8 | 2.3197 | 0.0030 |
| Example 6 | 20 | 20.64 | 0.226 | Preparation Example 6 | 5.0 | 238.2 | 2.3310 | 0.0031 |
| Example 7 | 20 | 20.64 | 0.226 | Preparation Example 7 | 5.0 | 218.1 | 2.3200 | 0.0030 |
| Example 8 | 20 | 20.64 | 0.226 | Preparation Example 8 | 5.0 | 226.3 | 2.3739 | 0.0033 |
| Example 9 | 20 | 20.64 | 0.226 | Preparation Example 9 | 5.0 | 231.4 | 2.2955 | 0.0028 |
| Example 10 | 20 | 20.64 | 0.226 | Preparation Example 10 | 5.0 | 226 | 2.2963 | 0.0028 |
| Example 11 | 20 | 20.64 | 0.226 | Preparation Example 11 | 5.0 | 220.7 | 2.3119 | 0.0029 |
| Comparative Example 1 | 20 | 20.64 | 0.226 | Comparative Preparation Example 1 | 5.0 | Not cured | | |
| Comparative Example 2 | 20 | 20.64 | 0.226 | Comparative Preparation Example 2 | 5.0 | 264.7 | 2.5503 | 0.0045 |

Referring to Table 2, the Examples prepared from the curing agent compositions including both the first compound and the second compound showed improved dielectric characteristics with reduced dielectric constant Dk and dissipation factor Df values compared to the Comparative Examples prepared from the curing agent compositions including only the second compound or only the third compound.

In addition, Examples 2 and 3 using the curing agent compositions additionally including the third compound showed an increase in the glass transition temperature Tg compared to Example 10 not including the third compound, thereby improving not only the dielectric characteristics but also the heat resistance characteristics.

The Examples and the Comparative Examples described above are illustrative examples for explaining the present disclosure, and the present disclosure is not limited thereto. Those skilled in the art will be able to implement the present disclosure through various modifications, and the technical protection scope of the present disclosure should be defined by the appended claims.

## Claims

1. A curing agent composition comprising:
a first compound represented by Formula 1; and
a second compound represented by Formula 2: 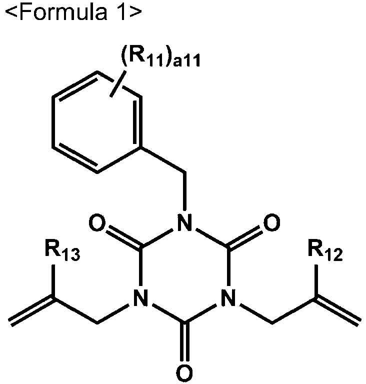 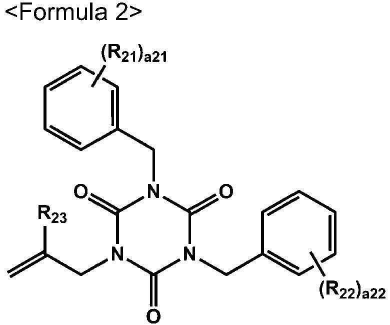
wherein, in Formulae 1 and 2,
R₁₁ to R₁₃ and R₂₁ to R₂₃ are each independently hydrogen, a C₁-C₁₅ alkyl group, or a C₂-C₁₅ alkenyl group, and
a11, a21, and a22 are each independently an integer from 0 to 5.

2. The curing agent composition of claim 1, wherein
an amount of the first compound is 70 to 99 weight% based on a total weight of the curing agent composition, and
an amount of the second compound is 1 to 30 weight based on the total weight of the curing agent composition.

3. The curing agent composition of claim 1, wherein
the curing agent composition has a decomposition temperature Td of 150 °C or more.

4. The curing agent composition of claim 1, wherein,
in Formula 1,
R₁₁ to R₁₃ are each independently hydrogen, a methyl group, or an ethyl group.

5. The curing agent composition of claim 1, wherein,
in Formula 2,
R₂₁ to R₂₃ are each independently hydrogen, a methyl group, or an ethyl group.

6. The curing agent composition of claim 1, further comprising
a third compound represented by Formula 3: 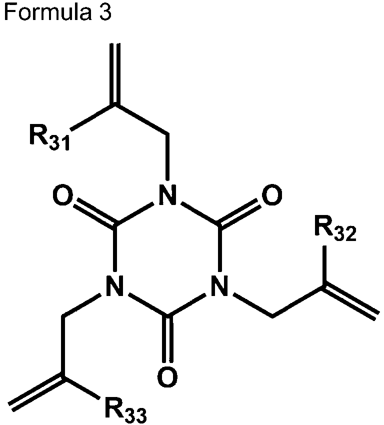
wherein, in Formula 3,
R₃₁ to R₃₃ are each independently hydrogen, a C₁-C₁₅ alkyl group, or a C₂-C₁₅ alkenyl group.

7. The curing agent composition of claim 6, wherein,
in Formula 3, R₃₁ to R₃₃ are each independently hydrogen, a methyl group, or an ethyl group.

8. The curing agent composition of claim 6, wherein
an amount of the third compound is 30 weight% or less based on a total weight of the curing agent composition.

9. A curing composition comprising:
the curing agent composition of claim 1;
a base polymer; and
an initiator.

10. The curing composition of claim 9, wherein
the base polymer comprises a polyphenylene oxide modified with acrylate, methacrylate, an aryl group, or a vinyl group.

11. The curing composition of claim 9, wherein
the initiator comprises a peroxide-based initiator.

12. The curing composition of claim 9, wherein
a ratio of the number of moles of the initiator to the number of moles of the curing agent composition is 0.01 to 0.5.

13. The curing composition of claim 9, wherein
a ratio of the weight ratio of the base polymer to the weight of the curing agent composition is 0.1 to 10.

14. The curing composition of claim 9, wherein
the curing composition has a dielectric constant Dk of 2.55 or less, and the curing composition has a dissipation factor Df of 0.0044 or less.
